# EUROPEAN PATENT SPECIFICATION

(11) **EP 1 311 637 B1**
(45) Date of publication and mention of the grant of the patent: **05.04.2006**
(21) Application number: 01962184.6
(22) Date of filing: 15.08.2001
(51) Int. Cl.: C09D 133/16, C08F 20/22, C08F 20/24

(54) **FLUOROALKYL (METH)ACRYLATE COPOLYMER COATING COMPOSITIONS**
FLUORO(METH)ACRYLATECOPOLYMER-BESCHICHTUNGSMASSEN
COMPOSITIONS DE REVETEMENT DE COPOLYMERE DE FLUOROALKYLE (METH)ACRYLATE

(30) Priority: 18.08.2000 US 226235 P
(43) Date of publication of application: 21.05.2003
(73) Proprietor: 3M Innovative Properties Company, St. Paul, MN 55133-3427 (US)
(72) Inventor: SAVU, Patricia, M., Maplewood, MN 55119 (US); FLYNN, Richard, M., Mahtomedi, MN 55115 (US); PARENT, Michael, J., Oakdale, MN 55128 (US)
(74) Representative: Vossius & Partner
(86) International application number: PCT/US2001/025527
(87) International publication number: WO 2002/016517

(56) References cited:
- EP-A- 0 889 092
- FR-A- 2 592 055

## Description

This invention pertains to polymerized fluoroalkyl (meth)acrylate coating compositions. In particular, this invention pertains to coating composition comprising fluoroalkyl (meth)acrylate/(meth)acrylic acid copolymer, wherein said copolymer comprises 5 wt.% or less of (meth)acrylic acid when the fluoroalkyl group is a perfluoroalkyl group and said fluoroalkyl group has 6 carbon atoms or fewer. More particularly, coating compositions are disclosed that are useful as anti-stiction coating for computer hard drives, barrier coatings to protect sensitive substrates such as circuit boards, and as antimigration coatings to prevent the migration of lubricants.

Fluorochemical compositions have achieved widespread use in a variety of applications, including, for example, in oil- and/or water-repellent compositions, and in surfactant compositions. Some known fluorochemical compositions ultimately degrade to perfluorooctyl-containing compounds when exposed to biological, thermal, oxidative, hydrolytic and photolytic conditions found in the environment. It has been reported that certain perfluorooctyl-containing compounds may tend to bio-accumulate in living organisms; this tendency has been cited as a potential concern regarding some fluorochemical compositions. For example, see U.S. Pat. No. 5,688,884 (Baker et al.). As a result, there is a desire for fluorine-containing compositions effective in providing desired functional properties, e.g., water- and oil- repellency, surfactant properties, etc. while eliminating more effectively from biological systems.

The present invention provides coating composition comprising fluoroalkyl (meth)acrylate/(meth)acrylic acid copolymers, wherein said copolymer comprises 5 wt.% or less, preferably 2 wt.% or less, (meth)acrylic acid and said fluoroalkyl group has 6 carbon atoms or fewer. The present coating compositions are surprising effective in providing low surface energy coatings for use in applications such as circuit board coatings, anti-stiction coatings, antimigration coatings and as films and coating used in microlithography pellicles.

The compositions of the invention, comprising fluoroalkyl acrylates (in which the fluoroalkyl group has less than six carbon atoms), ultimately degrade to the fluoroalkyl carboxylic acids (or salts thereof), which are believed to be more effectively eliminated that the perfluorooctyl-containing compounds.

The performance of the coating prepared from the present coating compositions are surprising in view of teachings that the lower perfluoroalkyl groups were significantly less effective than longer chain perfluoroalkyl groups, such as the perfluorooctyl group. For example, it has been demonstrated that surfactants derived from perfluorocarboxylic acids and perfluorosulfonic acids exhibit considerable differences in performance as a function of chain length. See, for example Organofluorine Chemicals and their Industrial Applications, edited by R. E. Banks, Ellis Horwood Ltd. (1979), p56; J. O. Hendrichs, *Ind. Eng Chem.,* 45, 1953, p103; M. K. Bemett and W. A. Zisman, *J*. *Phys. Chem.,* 63, 1959, p1912.

Further, various models have been devised to explain the low surface energies and resultant high contact angle data of fluorinated polymer coatings that are based on a monolayer of a fluorinated carboxylic or sulfonic acids present at the air/liquid interface. The data suggest that only after the seven outermost carbon atoms of the fluoroalkyl group (C₇F₁₅-) were fully fluorinated did the contact angles (and therefore the surface energies) of various liquids on the surface approach those of a perfluorinated acid monolayer (see N. O. Brace, *J*. *Org. Chem., 27,* 1962, p 4491 and W. A. Zisman, Advan. Chem, 1964, p 22.). Therefore, one would expect that the performance of fluorinated coatings containing fluoroalkyl groups (e.g., coatings made by polymerizing fluoroalkyl (meth)acrylates) could be predicted from the known performance of perfluorocarboxylic and perfluorosulfonic acid derivatives, and the surface energy of the fluoropolymer coatings would be related to the chain length of the fluoroalkyl group of the fluoropolymer coating.

Surprisingly, it has been found that the coatings comprising the fluoroalkyl (meth)acrylates having fluoroalkyl groups of six carbon atoms or less exhibit surface energies and contact angle performance which are comparable to coating comprising fluoroalkyl (meth)acrylates of the longer chain homologues, such as (meth)acrylates having perfluorooctyl groups.

The present coating compositions provide additional advantages. First, the precursor compounds containing the shorter fluoroalkyl groups useful in the coating compositions of the invention may be produced at a lower cost per weight because of higher yields while maintaining their potency as effective low surface energy coatings at the same weight basis. For example, the heptafluorobutyryl fluoride precursor may be prepared in yields of 60% as compared to perfluorooctanoyl fluoride precursor (31%) in an electrochemical fluorination process (Preparation Properties and Industrial Applications of Organofluorine Compounds, edited by R. E. Banks, Ellis Horwood Ltd (1982), p 26). Furthermore, the short chain carboxylic acids (the presumed intermediate degradation products) are less toxic and less bioaccumulative than the longer chain homologues.

The fluoroalkyl (meth)acrylate/(meth)acrylic acid copolymer (hereinafter the fluorocopolymer) contains less than 1 wt% of acrylic acid or methacrylic acid and is preferably a random copolymer of the fluoroalkyl (meth)acrylate and (meth)acrylic acid. Incorporating a small amount of (meth)acrylic acid into the fluorocopolymer improves the adhesion of the coating to polar substrates, such as the metallic surfaces of computer hard drives or optical pellicle frames, and optimizes both the low surface energy properties and refractive indices of the fluorocopolymer. The presence of greater than 5 wt.% of (meth)acrylic acid in the fluorocopolymer may unduly raises both the surface energy and refractive index, and often results in a hazy coating, rendering it unacceptable for uses where optical clarity is desired. Further it has been found that elevated levels of (meth)acrylic acid lack the requisite solubility in the preferred solvents.

As used herein (meth)acrylate refers to both acrylates and methacrylates, and (meth)acrylic acid refers to both acrylic acid and methacrylic acid.

The fluorocopolymer is of the formula 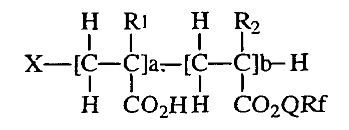 wherein the sum of a + b is an number such that the compound is polymeric, preferably a + b is such that the number average molecular weight, Mₙ is from 5,000 to 20,000 with M_{w}/Mₙ = 2 to 3;
R₁ is a hydrogen atom or a methyl group;
R₂ is a hydrogen atom or is a straight chain or branched chain alkyl group containing 1 to about 4 carbon atoms;
Q is a covalent bond or an organic divalent linking group,
R_{f} is a fluoroaliphatic group that comprises a fully- or partially-fluorinated terminal group having less than 6 carbon atoms;
and X is a hydrogen atom or a group derived from a free radical initiator.

A salient component of the fluorocopolymer is the fluoroalkyl group, designated herein as R_{f}. The fluorinated compound of the invention contains a plurality of pendent R_{f} groups (e.g., from 2 to about 10) proximal to one another and preferably contains from about 5 percent to about 80 percent, more preferably from about 20 percent to about 65 percent, and most preferably about 25 percent to about 55 percent fluorine by weight, based on the total weight of the compound, the loci of the fluorine being essentially in the R_{f} groups. R_{f} is a stable, inert, nonpolar, preferably saturated, monovalent moiety which is both oleophobic and hydrophobic. R_{f} preferably contains at least about 3 carbon atoms, more preferably 3 to about 5 carbon atoms, and most preferably 3 or 4 carbon atoms. R_{f} can contain straight chain, or branched chain. R_{f} is preferably free of polymerizable olefinic unsaturation. It is preferred that R_{f} contain about 35% to about 78% fluorine by weight, more preferably about 40% to about 78% fluorine by weight. The terminal portion of the R₁ group contains a fully fluorinated terminal group, eg -CF₃, or may be partially fluorinated, e.g. HCF₂-.

Preferably the (meth)acrylate is derived from an alcohol selected from CF₃CHFCF₂CH₂OH, HCF₂CF₂CH₂OH, C₂F₅CH(OH)CF(CF₃)₂, C₃F₇CH₂OH, and C₄F₉CH₂OH.

Preferably Q is a divalent linking group of the formula -CₙH₂ₙ-, where n is an integer of 1 to 4; most preferably Q is -CH₂-. With respect to the formula, a and b are numbers such that a and b may represent non-integral values, or averages of the number of each monomer unit present. Preferably a and b are numbers such that the fluorocopolymer has a molecular weight Mₙ of from about 5,000 to 20,000 with a M_{w}/Mₙ ranging from 2 to 3.

The fluorochemical (meth)acrylates and fluorocopolymers may be prepared as described in U.S. Pat. Nos. 2,803,615 (Ahlbrecht et al.) and 2,841,573 (Ahlbrecht et al.) which disclosures are incorporated herein by reference. Preferred fluorochemical (meth)acrylates includes 1,1-dihydroperfluorobutyl (meth)acrylate, 1,1-dihydropentafluoropropyl (meth)acrylate and hexafluoroisopropyl (meth)acrylate.

If desired, the coating composition may further comprise a solvent. Although any conventional solvent, including both fluorinated and non-fluorinated solvents, it is preferred that the solvents be selected that are non-ozone depleting, non-flammable and fast drying. Fluorinated solvents are preferred. Among fluorinated solvents, partially fluorinated solvents are more preferred over perfluorinated solvents due to the long atmospheric lifetime of the perfluorocarbons. The partially fluorinated solvents includes hydrochlorofluorocarbons (HCFCs), hydrofluorocarbons (HFCs) and hydrofluoroethers (HFEs).

The term "hydrochlorofluorocarbon" means compounds consisting only of the elements carbon, hydrogen, fluorine and chlorine. Useful HCFCs include CF₃CHCl₂, CH₃CCl₂F, CF₃CF₂CHCl₂ and CClF₂CF₂CHClF.

The term "hydrofluorocarbon" means compounds consisting only of the elements carbon, hydrogen and fluorine. The carbon backbone of the HFC can be straight, branched, cyclic, or mixtures of these, and can contain from 3 to 8 carbon atoms. Useful HFCs include compounds having more than approximately 5 molar percent fluorine substitution, or less than 95 molar percent fluorine substitution, based on the total number of hydrogen and fluorine atoms bonded to carbon, such compounds including CF₃CFHCFHCF₂CF₃, C₅F₁₁H, C₆F₁₃H, CF₃CH₂CF₂CH₃, CF₃CF₂CH₂CH₂F, CHF₂CF₂CF₂CHF₂, 1,2-dihydroperfluorocyclopentane and 1,1,2-trihydroperfluorocyclopentane. Useful HFCs are available under the VERTREL™, available from E. I. duPont de Nemours & Co. and under the ZEORORA-H™, available from Nippon Zeon Co. Ltd., Tokyo, Japan.

The term "hydrofluoroether", in connection with the present invention, means compounds that contain carbon, hydrogen, fluorine and at least one ether oxygen, and are free of chlorine, bromine and iodine. Hydrofluoroethers useful in this invention are typically liquid at ambient temperature and pressure (about 20°C and 760 torr), are non-ozone depleting and can be non-flammable. Non-flammable hydrofluoroethers are preferred as solvents or dispersing media in this invention. In connection with this invention, the term "non-flammable hydrofluoroethers" means the hydrofluoroethers which do not exhibit a flash point when tested in a closed cup flash point test performed according to ASTM D 56-87.

One useful class of hydrofluoroethers include alpha-, beta- and omega- substituted hydrofluoroalkyl ethers such as those described in U.S. Patent No. 5,658,962 (Moore et al.), incorporated herein by reference, and those described by Marchionni et al. in "Hydrofluoropolyethers," Journal of Fluorine Chemistry 95 (1999), pp. 41-50, which can be described by the general structure shown in Formula I:

X-[R_{f}' - O]_{y}R"H (I)

wherein:
X is either F, H, or a perfluoroalkyl group containing from 1 to 3 carbon atoms which is optionally hydro-substituted in the omega position;
each R_{f}' is independently selected from the group consisting of -CF₂-, -C₂F₄-, and -C₃F₆-, wherein, when X is perfluorinated, X and at least a portion of the adjacent R_{f}' group taken together can form a perfluorocycloalkyl group;
R" is a divalent organic radical having from 1 to about 3 carbon atoms, and may be perfluorinated, unfluorinated or partially fluorinated; and
y is an integer from 1 to 7;
wherein when X is F, R" contains at least one F atom; and wherein preferably the total number of carbon atoms is between about 3 and about 8.

Representative compounds described by Formula VIII useful in the present invention include, but are not limited to, the following compounds:

C₄F₉OC₂F₄H

HC₃F₆OC₃F₆H

HC₃F₆OCH₃

C₅F₁₁OC₂F₄H

C₆F₁₃OCF₂H

C₃F₇OCH₂F

HCF₂OCF₂OCF₂H

HCF₂OCF₂OC₂F₄OCF₂H

HCF₂OC₂F₄OC₂F₄OCF₂H

C₃F₇O[CF(CF₃)CF₂O]ₚCF(CF₃)H,

wherein p = 0 to 1

HCF₂OC₂F₄OCF₂H

HCF₂OCF₂OCF₂OCF₂H

HCF₂OC₂F₄OC₂F₄OCF₂H

c-C₆F₁₁OCF₂H

c-C₆F₁₁OCH₂F

Preferred non-flammable, non-segregated (i.e. having fluorine atoms on both sides of the ether oxygen atom) HFEs include C₄F₉OC₂F₄H, C₆F₁₃OCF₂H, HC₃F₆OC₃F₆H, C₃F₇OCH₂F, HCF₂OCF₂OCF₂H, HCF₂OC₂F₄OCF₂H, HCF₂OCF₂OCF₂OCF₂H, HCF₂OCF₂CF₂OCF₂H, HC₃F₆OCH₃, HCF₂OCF₂OC₂F₄OCF₂H, and mixtures thereof. Non-segregated HFEs are available from Ausimont Corp., Milano, Italy, under the GALDEN H™.

Particularly preferred hydrofluoroethers are perfluoroalkyl alkyl ethers and more preferably, the hydrofluoroethers correspond to the Formula II:

(R-O)ₓ-R'_{f} (II)

wherein x is 1 or 2; R represents an alkyl group having about 1 to 4 carbon atoms; and R'_{f} represents a fluoroaliphatic group.

R'_{f} preferably contains between about 2 to 9 carbon atoms and, when x is 1, is preferably selected from the group consisting of a linear or branched perfluoroalkyl group, perfluorocycloalkyl group-containing perfluoroalkyl groups, perfluorocycloalkyl groups, linear or branched perfluoroalkyl groups having one or more catenary atoms, perfluorocycloalkyl-group-containing perfluoroalkyl groups having one or more catenary heteroatoms (e.g. N, O or S) and perfluorocycloalkyl group having one or more catenary heteroatoms and, when x is 2, is preferably selected from the group consisting of a linear or branched perfluoroalkylene groups, perfluorocycloalkyl group-containing-perfluoroalkylene groups, perfluorocycloalkylene groups, linear or branched perfluoroalkylene groups having one or more catenary atoms, perfluorocycloalkyl group-containing perfluoroalkylene groups having one or more catenary heteroatoms and perfluorocycloalkylene groups having one or more catenary heteroatoms.

More preferably, x is 1, and the compound is normally liquid. R'_{f} is selected from the group consisting of linear or branched perfluoroalkyl groups having from 3 to about 9 carbon atoms, perfluorocycloalkyl-containing perfluoroalkyl groups having from 5 to about 7 carbon atoms, and perfluorocycloalkyl groups having from 5 to about 6 carbon atoms; R is a methyl or ethyl group; R'_{f} can contain one or more catenary oxygen or nitrogen heteroatoms; and the sum of the number of carbon atoms in R'_{f} and the number of carbon atoms in R is greater than or equal to about 4. Such hydrofluoroethers are especially preferred in the coating composition of the invention due to the non-flammability, zero ozone depletion, low global warming potential, ease of evaporation from the coating, and solubility of the fluorocopolymer in the hydrofluoroether. It is further preferred that the fluorocopolymer be prepared in the hydrofluoroether to avoid the necessity of removing another polymerization media solvent.

Representative examples of hydrofluoroethers suitable for use in the processes and composition of the invention include the following compounds : n-C₄F₉OCH₃, n-C₄F₉OCH₂CH₃, CF₃CF(CF₃)CF₂OCH₃, CF₃CF(CF₃)CF₂OC₂H₅, C₈F₁₇OCH₃, CH₃O-(CF₂)₄-OCH₃, C₅F₁₁OC₂H₅, C₃F₇OCH₃, CF₃OC₂F₄OC₂H₅, C₃F₇OCF(CF₃)CF₂OCH₃, (CF₃)₂CFOCH₃, C₄F₉OC₂F₄OC₂F₄OC₂H₅, C₄F₉O(CF₂)₃OCH₃, and 1, 1 -dimethoxyperfluorocyclohexane.

The compositions of the present invention are useful as antimigration coatings to prevent the spreading of a lubricant beyond the desired areas of lubrication. Typically the lubricants used in contacting parts must be confined or otherwise contained to prevent the lubricant from spreading. The undesired spreading or migration of the lubricants typically depletes the lubricant, resulting in catastrophic failure of the device. The coatings of the present invention, having critical surface tensions typically less than 15 dynes/cm, prevent or retard the migration of lubricants which have critical surface tensions greater than these values, so the lubricant does not wet the anti-migration coating. The barrier coating is of a size and shape, adjacent to the area of lubrication, sufficient to prevent migration of the lubricant, and may surrounds the lubricant and therefor containing it in the desired area of lubrication. For example, the fluorocopolymer could be coated in the shape of a ring surrounding a quantity of lubricant in contact with the lubricated surfaces. Any suitable lubricants may be used, including silicones, petroleum oils and greases, aliphatic diester oils and polyether oils. The antimigration coatings may be used to contain other fluids subject to migrations, such as inks and fluxes.

Thus the present invention provides a method for containing a lubricant at the area of frictional contact between parts of a mechanical assembly by providing a coating of the present invention in a predetermined pattern near the area of frictional contact arranged so as to prevent the lubricant from spreading beyond the area of frictional contact. The anti-migration coating may be used with any suitable mechanical assembly having parts in frictional contact, including motors, gears, actuators or wherever it is desirable to prevent migration of lubricant.

It has been found that electronic components on printed circuit boards may be coated with the present coating composition to insulate them from contaminants and preserve the electronic functions. Such coatings are thermally and chemically stable and protect the circuit boards from atmospheric moisture.

The circuit board may be coated before the electronic components have been mounted to protect the component from moisture, dirt, oil, dust or other contaminants, though generally the components are mounted prior to coating. Useful electronic components include circuit chips, transistors, resistors, capacitors, thermistors, thyristors, diodes and the like. The electronic components are generally mounted in electronic connection to the circuit using any suitable means such as soldering, welding, ultrasound and other methods that bond by heating the two surfaces in frictional contact.

Thus the present invention provides a method for forming a protective coating on a circuit board by applying the coating composition of the present invention to at least a portion of the circuit board, and evaporating any solvent present in the coating composition. The invention further provides a circuit board having a protective coating comprising the coating composition of the invention.

The present invention may also provides anti-stiction coating for magnetic recording heads and micromechanical devices such as micromotors, microgears and deformable mirror devices. Stiction is the tendency of a landed, stationary device such as a magnetic recording head, to resist translational movement from a surface such as a magnetic disk. High stiction can lead to mechanical failure of the device upon start up.

In magnetic disk drives, sliders are employed to aerodynamically fly a magnetic transducer over the surface of the disk. The slider forms a pressure region to develop the desired pressure between the slider and disk surface to achieve the desired flying characteristics for the slider. One problem associated with air-bearing sliders is that the liquid lubricant on the surface of the disk can transfer to the slider by wicking when the media has stopped spinning and the slider rests on the disk. This wicking, along with the accumulation of other debris such as dust, increase stiction between the slider and the disk, thus requiring additional power to take off the slider. Additionally any lubricant that wicks to the surface of the slider, and any debris which collects thereon, can adversely affect the flying characteristics of the slider.

Many of these problems may be overcome by applying a thin film of the present coating composition to the surface of the slider. The resulting coating has a very low surface energy and resists wicking of the disk lubricant to the surface of the slider, and further resists the accumulation of other debris such as dust particles. The thin film may be applied to the slider by any conventional means such as dipping the slider into the coating composition, and curing (if necessary) the deposited coating composition to form on the slider a thin film of generally less than 15 Angstroms thickness. Thus the present invention provides an improved slider for use in a magnetic media disk, the slider bearing a thin film of the coating composition of the invention.

The coating composition of the present invention may also be used to produce pellicles used in microlithography. In the semiconductor chip industry it is known that a pattern transfer from a photomask to a substrate is accomplished by exposing the mask to a light source. During the pattern transfer process (the photolithographic process), patterns on the photomask are projected onto the substrate that has been treated with a photosensitive substance. This results in the mask pattern being transferred to the substrate. Unfortunately, any foreign substance on the surface of the mask will also be reproduced on the substrate and will therefore interfere with proper pattern transfer.

To eliminate contamination of the mask surface, a framed thin membrane called a pellicle in mounted on the mask surface such that the pellicle film extends co-planer to the mask, and is spaced a predetermined distance from the mask surface by means of a pellicle frame. Any contamination that would ordinarily land on the mask instead falls on the pellicle film. Contamination on the pellicle will not generally be projected onto the substrate. The contamination may be removed from the pellicle surface, or may be adjusted to be out of focus during pattern transfer.

The pellicle must be highly transparent in the region of the incident light, and clean in order to meet the rigid processing standards. The pellicle film should be securely mounted to the frame to maintain uniform tension across the surface of the film. Generally, the film is adhesively secured to the frame. Generally the pellicle film is less than 5 micrometers in thickness, and of predetermined area, depending on the dimensions of the substrate.

In recent years photolithography has used UV light with wavelengths less than 300 nanometers to fabricate devices of increasingly small size. Pellicles for use with so-called deep UV are generally thinner, normally less than 2 micrometers and often less than 1.2 micrometers, in order to obtain maximum transmission through the pellicle film at wavelengths such as 248 nanometers.

The present invention provides pellicles for use in photolithography comprising a film of the instant fluorocopolymer, said film being secured to a frame. Generally the fluorocopolymer film is adhesively secured to the frame. Suitable adhesives are described in, for example, U.S. 5,772,817 (Yen et al.) incorporated herein by reference. Generally the pellicle film comprises a film of the present fluorocopolymer less than 5 micrometers, and preferably less than 1.2 micrometers. If desired, the pellicle film may comprise a coating of the fluorocopolymer on a conventionally pellicle film such as nitrocellulose or other cellulosic polymer. A freestanding film of the fluorocopolymer may be prepared by any conventional technique such a blown films, or by casting either neat or from a suitable solvent. When the pellicle comprises a coating of the fluorocopolymer on a cellulosic film, it too may be prepared by coating a solution of the fluorocopolymer onto an extant cellulosic film, or by coextrusion of the two film layers.

The low surface energy of the fluorocopolymer makes it easier to remove contaminants, such as dust, from the pellicle, by brushing or blowing. Additionally, the fluorocopolymers are not subject to the build-up of static charges that attract and hold particulate contaminants on the pellicle surface.

The present pellicles enjoy other advantages due to the low refractive index of the fluorocopolymer. When light passes from a medium of one refractive index to another, some surface reflection occurs which results in a loss of transmittance. Air has a refractive index of 1.0002 (Handbook of Chemistry and Physics, 56^{th} Edition, CRC Press, 1975,E-224). The refractive indices of conventional cellulosic polymers are 1.48-1.5 and 1.5-1.51 (Polymer Handbook, Fourth. Edition, Editors J. Brandrup, E. H. Immergut, E. A. Grulke, John Wiley and Sons,1999, pVI/578). The refractive indices of the fluorocopolymers of the present invention are generally lower, typically in the range of 1.339 to 1.437.

The reflectance of an interface between two non-absorbing media of refractive indices n₁ and n₂ is given by the equation R=[(n₁- n₂)/ (n₁+n₂)]².

Of the incident light only a fraction (1-R)² passes through the pellicle without undergoing any reflections (Handbook of Optics, Vol 1, Michael Bass, editor, McGraw Hill, 1995, p42.19). Only the light that passes through the pellicle can initiate polymerization of the mask polymer that will become the circuit path after etching. Using an antireflection coating of the low refractive index fluorocopolymer on the pellicle will reduce reflections and increase the transmission of light incident on the mask.

For any desired application, the coating composition may be coated neat or delivered from any suitable solvent in which the fluorocopolymer dissolves to a suitable degree, such solvent not deleteriously affecting the substrate (such as a circuit board or mounted components) and not leaving a harmful residue, such as a conductive residue. Typically the coating composition may comprise up to about 50 weight % fluorocopolymer, though it is preferred the composition comprise 20 weight % or less, and most preferably 10 weight % or less. The resultant coatings are generally less than about 10 mils thick, depending on the application, although thicker coatings may be prepared. The coating composition may be applied to the substrate using any suitable coating means known in the art including, but not limited to, brushing, dipping, spraying, etc. If a solvent is present the composition may be cured or dried by evaporation, aided by heat, if desired.

### EXAMPLES

The present invention will be further described with reference to the following non-limiting examples. All parts, percentages and ratios are by weight unless otherwise specified.

### Fluorocopolymer 1. 2,2,3,3,4,4,4-Heptafluorobutyl Methacrylate (CF₃CF₂CF₂CH₂OC(O)C(CH₃)=CH₂)/Acrylic Acid Copolymer (99.5/0.5)

9.95 g of 2,2,3,3,4,4,4-heptafluorobutyl methacrylate (FBMA), 0.05 g of acrylic acid, 0.3 g of LUPEROX™ 26-M60 (available from Elf Atochem North America, Philadelphia, PA), and 57 g of NOVEC™ HFE-7100 Engineering Fluid (methyl perfluorobutyl ether, available from 3M Co., St. Paul, MN) were placed in a reaction vessel and purged with dry nitrogen. The vessel was sealed and the mixture was heated with agitation at 80°C for 18 hours. After 18 hours, the vessel was cooled and opened. Then 4.1 g of mixture was withdrawn from the vessel and was heated in a small pan for 30 minutes in an oven set at 125°C to give 0.62 g of clear hard fluoropolymer.

The FBMA was prepared as follows. In a 3 liter flask equipped with overhead stirrer, thermocouple and addition funnel were placed 1260 g of heptafluorobutanol, 20 g of 95% sulfuric acid, 1.2 g of phenothiazine and 1 g of 4-methoxyphenol (MEHQ). The reaction mixture was heated to 55°C, and then the addition of 946 g of methacrylic anhydride was begun. The reaction mixture exothermed to 65°C, so the addition rate was adjusted to keep the reaction mixture temperature at 65°C. The addition of methacrylic anhydride was completed in 2.5 hours. The reaction mixture was heated at 65°C for an additional 3 hours, then was allowed to cool to room temperature. 1200 mL of deionized water was added and the resulting reaction mixture was stirred for 30 minutes. The mixture was allowed to phase-split, and the translucent amethyst-colored fluorochemical lower phase was saved. The lower phase was then stirred for 30 minutes with a mixture of 416 g of sodium carbonate, 50 g of sodium hydroxide and 1500 g of water. Again, the mixture was allowed to phase-split, and the lower fluorochemical phase was saved and washed twice with 1500 g aliquots of water to give 1531 g of crude FBMA. The crude methacrylate was added to a 3 liter flask fitted with a distillation head and a thermocouple. More polymerization inhibitors (3 g of phenothiazine and 0.7 g of MEHQ) were added to the distillation flask. The acrylate was distilled to give 138 g of precut distilling at 176 torr at a head temperature of 61-79°C. The precut was then distilled at 79-85°C and 161 torr to give a total of 1274 g of purified FBMA.

### Fluorocopolymer 2. 2,2,3,4,4,4-Hexafluorobutyl Acrylate (CF₃CFHCF₂CH₂OC(O)CH=CH₂)/Acrylic Acid Copolymer (98/2)

In a flask equipped with overhead stirrer, thermocouple and addition funnel were placed a solution of 30.3 g (0.121 mol) of 2,2,3,4,4,4-hexafluorobutyl acrylate (available from PCR) and 0.57 g (0.0079 mol) of acrylic acid dissolved in 175 g of NOVEC™ HFE-7200 Engineering Fluid (ethyl perfluorobutyl ether, available from 3M Co.). Then LUPEROX™ 26-M50 initiator (1.23 g of 50% solution in mineral spirits, available from Elf Atochem) was added and the resulting solution was purged with dry nitrogen. The solution was then heated for 16 hours at 73°C under dry nitrogen, during which time the polymer precipitated. The HFE-7200 was decanted and the polymer was allowed to air dry for two hours at ambient lab conditions. The polymer was insoluble in HFE-7200 and HFE-71 00, but the polymer readily dissolved in NOVEC™ HFE-71IPA Engineering Fluid (a 95/5 mixture of HFE-7100 and isopropanol, available from 3M Co.) to form a 7.9% solution.

### Fluorocopolymer 3. 2,2,3,3-Tetrafluoropropyl Methacrylate (HCF₂CF₂CH₂OC(O)C(CH₃)=CH₂)/Methacrylic Acid Copolymer (97.5/2.5)

In a flask equipped with overhead stirrer, thermocouple and addition funnel were placed a solution of 25 g (0.125 mol) of 2,2,3,3-tetrafluoropropyl methacrylate (prepared by reaction of HC₂F₄CH₂OH with methacrylic anhydride using essentially the same procedure as described in Example 1) and 0.64 g (0.0074 mol) of methacrylic acid dissolved in 145 g of HFE-7200. Then LUPEROX™ 26-M50 initiator (1.02 g of 50% solution in mineral spirits) was added and the polymerization was carried out as described in Example 3. The polymer completely precipitated during the polymerization process and was filtered, air dried and then dissolved in MIBK to afford a 10% solution. The glass transition temperature (T_{g}) for this polymer was 68ºC.

### Fluorocopolymer 4. 2,2,3,3,4,4,4-Heptafluorobutyl Methacrylate (CF₃CF₂CF₂CH₂OC(O)C(CH₃)=CH₂)/Acrylic Acid Copolymer (99/1)

19.8 g of heptafluorobutyl methacrylate (made as described for Fluorocopolymer 1), 0.2 g of acrylic acid, 0.4 g of LUPEROX™ 26M60 initiator and 113 g of NOVEC™ HFE-7100 Engineering Fluid were place in a vessel, and the vessel and its contents were purged with dry nitrogen. The vessel was sealed and the contents heated with agitation at 60°C for 18 hours. After 18 hours, the vessel was cooled and opened. 4.2 g of material was withdrawn and heated in a small pan in the oven at 125°C for 30 minutes to give 0.57 g of clear hard fluorocopolymer.

### Fluorocopolymer 5. 1,1-Dihydroperfluorocyclohexylmethyl Methacrylate (c-C₆F₁₁CH₂OC(O)C(CH₃)=CH₂)/Acrylic Acid Copolymer (99/1)

First, 1,1-dihydroperfluorocyclohexylmethyl methacrylate was prepared. A 3 liter round-bottomed flask, equipped with an overhead stirrer and dropping funnel, was charged with 840 g of trifluoroacetic anhydride (available from Aldrich Chemical Co., Milwaukee, WI). The flask and its contents were immersed into an ice-water bath (<5° C). 380 grams of methacrylic acid (available from Aldrich Chemical Co.) was added to the flask over a period of 10 minutes. The reaction mixture was then stirred for approximately 30 minutes at ice-water temperature. The cooled reaction mixture was then charged with 1000 g of 1,1-dihydroperfluorocyclohexylmethyl alcohol (the alcohol can be prepared by the general procedure described in U.S. Pat. No. 2,666,797). The resulting mixture was stirred for approximately 30 minutes at ice-water temperature followed by approximately 16 hours at room temperature.

Crude fluorochemical methacrylate monomer was isolated by adding the reaction mixture to 1 liter of water cooled to <5° C (i.e., immersion in an ice-water bath). The resulting two phases (aqueous and organic) were separated. The organic phase was washed twice with 500 mL aliquots of deionized water, twice with 500 mL aliquots of 0.2N sodium hydroxide, and finally with 500 mL aliquots of saturated aqueous sodium chloride solution. The washed organic phase was dried over anhydrous magnesium sulfate.

The dried residue, containing crude fluorinated monomer product, was purified by column chromatography using silica gel (available from Keiselgelä 60, Merck, Poole, England) as the stationary phase and 5% diethyl ether in 30°-40° C. petroleum ether as the eluent. The solvents were removed under reduced pressure, and the crude fluorinated monomer residue was vacuum distilled at 9 torr to leave pure 1,1-dihydroperfluorocyclohexylmethyl methacrylate, which was stored at 5° C prior to use.

The 99/1 copolymer of 1,1-dihydroperfluorocyclohexylmethyl methacrylate/acrylic acid was prepared using essentially the same procedure as described for Fluorocopolymer 1 except that an equal weight of 1,1-dihydroperfluorocyclohexylmethyl methacrylate was substituted for the 2,2,3,3,4,4,4-heptafluorobutyl methacrylate.

### Fluorocopolymer 6. 2,2,3,3,4,4,4-Heptafluorobutyl Methacrylate (CF₃CF₂CF₂CH₂OC(O)CH-CH₂)/Acrylic Acid Copolymer (90/10)

Fluorocopolymer 6 was prepared using essentially the general procedure as described for the preparation of Fluorocopolymer 1. In this case, 45 g (0.168 mol) of 2,2,3,3,4,4,4-heptafluorobutyl methacrylate (FBMA) was copolymerized with 5 g (0.069 mol) of acrylic acid in 50 mL of methyl ethyl ketone using 0.25 of VAZO™ 64 initiator (2,2'-azobisisobutyronitrile, available from E. I duPont de Nemours & Co., Wilmington, DE) by heating the reaction mixture to 65°C for 17 hours. The resulting polymer had a glass transition temperature (T_{g}) of 71.3°C.

### Fluorocopolymer 7. 2,2,3,3,4,4,4-Heptafluorobutyl Methacrylate (CF₃CF₂CF₂CH₂OC(O)C(CH₃)=CH₂)/Methacrylic Acid Copolymer (90/10)

Fluorocopolymer 7 was prepared using essentially the general procedure as described in Example 1 of U.S. Pat. No. 4,849,291. In this case 45 g (0.168 mol) of 2,2,3,3,4,4,4-heptafluorobutyl methacrylate was copolymerized with 5 g (0.058 mol) of methacrylic acid in 50 mL of methyl ethyl ketone using 0.25 of VAZO™ 64 initiator by heating the reaction mixture to 65°C for 17 hours. The resulting polymer had a glass transition temperature (T_{g}) of 109.1 °C.

### Fluorocopolymer 8. 2,2,3,3,4,4,4-Heptafluorobutyl Methacrylate (CF₃CF₂CF₂CH₂OC(O)C(CH₃)=CH₂)/Acrylic Acid Copolymer (99.95/0.05, higher molecular weight)

1120.0 g of 2,2,3,3,4,4,4-heptafluorobutyl methacrylate (FBMA), 5.6 g of acrylic acid (AA), 45.0 g of LUPEROX™ 26M50 initiator (to give a ratio of initiator to fluorochemical monomer of 0.04) and 6400 g of HFE-7100 were placed in a 2 gal (7.6 L) Parr reactor and purged of oxygen by pressurizing with dry nitrogen, followed by evacuation several times to the vapor pressure HFE-71 00. The vessel was sealed and the mixture was heated with agitation at 80°C for 23 hours to complete the reaction and provide a 15% solution.

The molecular weight distribution of the resulting polymer was measured using gel permeation chromatography (gpc). From the distribution, the number average molecular weight (Mₙ), the weight average molecular weight (M_{w}) and the average molecular weight (M_{z}) were determined, and the M_{w}/Mₙ ratio, representing molecular weight polydispersity, was calculated. Those values were as follows:
Mₙ = 10400
M_{w} = 22300
M_{z} = 35400
M_{w}/Mₙ = 2.1

### Fluorocopolymer 9. 2,2,3,3,4,4,4-Heptafluorobutyl Methacrylate (CF₃CF₂CF₂CH₂OC(O)C(CH₃)-CH₂)/Acrylic Acid Copolymer (99.95/0.05, lower molecular weight)

For Fluorocopolymer 9, essentially the same procedure was followed as in the preparation of Fluorocopolymer 8 except that the amounts of reactants employed were 805.1 g of FBMA, 4.0 g of AA, 56.4 g of LUPEROX™ 26M50 initiator (to give a ratio of initiator to fluorochemical monomer of 0.07) and 6400 g of HFE-7100.

The molecular weight information recorded for the resulting fluorocopolymer, having the same composition as Fluorocopolymer 10 but having lower molecular weight and greater polydispersity, are shown below:
Mₙ = 6180
M_{w} = 16700
M_{z}= 35100
M_{w}/Mₙ = 2.7

### Contact Angle Measurement Test Procedure

A good reference for contact angle measurements is "Measurement of Interfacial Tension and Surface Tension - General Review for Practical Man," GIT Fachzeitschrift für Das Laboratorium, 24 (1980), pp. 642-648 and 734-742), G-I-T Verlag Ernst Giebeler, Darmstadt.

Advancing and receding contact angle measurements were made with a Krüss Processor Tensiometer, Model K-12 (available from Krüss KmbH, Hamburg, Germany), using the Wilhelmy Plate Method.

For advancing contact angle measurements, the sample was suspended, from above the platform, the reservoir on the platform was filled with either n-hexadecane or water, and the platform was raised toward the suspended sample until the sample was completely submerged. The advancing contact angle was calculated by the instrument as the sample was being submerged.

For receding contact angle measurements, the reverse procedure was followed as with the advancing contact angle measurements, that is, the suspended sample was first immersed in the n-hexadecane or water, then the platform was lowered so that the sample was gradually pulled out of the test liquid. The receding contact angle was calculated by the instrument as the sample was being pulled out.

Static contact angles were measured using a Rame-Hart Model 100-00115 goniometer, available from Rame-Hart, Mountain Lakes, NJ.

### Examples 1-2 and Comparative Example C1

In Examples 1 and 2, Fluorocopolymers 1 and 2, both fluorocopolymers of this invention, were compared in repellency to the fluorocopolymer in 3M FLUORAD™ FC-732 Fluorochemical Coating (Comparative Example C1). FC-732 is a 2% solution of a copolymer of 99/1 C₇F₁₅CH₂OC(O)C(CH₃)=CH₂/CH₂=CHC(O)OH in methyl perfluorobutyl ether (available as NOVEL™ HFE-7100 engineering fluid from 3M Company, St. Paul, MN). To run the tests, each fluorocopolymer solution was diluted with HFE-7100 to 0.2% solids solution, a test wafer were dipped into each diluted fluorocopolymer test solution, each wafer was allowed to dry under ambient conditions, and advancing (Adv), static (Stat) and receding (Rec) contact angles were measured with n-hexadecane and using deionized water following the Contact Angle Test Procedure. For this test series, the test wafer used was a #310 3M Al₂O₃-TiC Ceramics wafer (available from 3M Company, cut to a size of ½ in (1.3 cm) x 3/4 in (1.9 cm) x 0.054 in (1.4 mm). Results from these contact angle measurements are presented in TABLE 1.

**TABLE 1**

| **Ex.** | **Fluorocopolymer** | ***n*-Hexadecane:** | | | **Water** | | |
|---|---|---|---|---|---|---|---|
| | | **Adv** | **Stat** | **Rec** | **Adv** | **Stat** | **Rec** |
| 1 | 1 | 66 | 62 | 45 | 118 | 107 | 75 |
| 2 | 2 | N/M* | 42 | N/M * | 104 | 95 | 67 |
| C1 | FC-732 | 51 | 60 | 39 | 125 | 108 | 45 |

| | | | | | | | |
|---|---|---|---|---|---|---|---|
| * Not measured | | | | | | | |

The data in TABLE 1 show that the fluorocopolymer of Example 1, containing a perfluorinated propyl (C3) terminal group (i.e., CF₃CF₂CF₂-), demonstrated essentially equivalent overall repellency to the fluorocopolymer of Comparative Example C1, which contains a perfluorinated heptyl (C7) terminal group (i.e., C₇F₁₅-). The fluorocopolymer of Example 2, containing a highly fluorinated propyl terminal group (i.e., CF₃CFHCF₂-), also demonstrated good repellency but slightly inferior to the fluorocopolymer of Example 1.

### Examples 3-9 and Comparative Example C2

In this series of experiments, the same general procedure was followed as with the tests run in TABLE 1, except that this time the test wafer was 6061 T-6 bare aluminum (available from Metaspec Co., San Antonio, TX, coupon size of 1 in (2.5 cm) x 1 in (2.5 cm) x 0.032 in (0.8 mm), polished, with hanging hole). All fluorocopolymers were applied at 2% solids from HFE-71 00. Results are presented in TABLE 2.

**TABLE 2**

| **Ex.** | **Fluorocopolymer** | ***n*-Hexadecane:** | | | **Water** | | |
|---|---|---|---|---|---|---|---|
| | | **Adv** | **Stat** | **Rec** | **Adv** | **Stat** | **Rec** |
| 3 | 1 | 65 | 66 | 32 | 118 | 107 | 61 |
| 4 | 2 | 66 | 67 | 35 | 118 | 110 | 62 |
| 5 | 3 | 65 | 62 | 38 | 98 | 95 | 66 |
| 6 | 4 | 66 | 67 | 35 | 118 | 110 | 62 |
| 7 | 5 | 62 | 65 | 40 | 123 | 108 | 54 |
| 8 | 6 | 67 | 61 | 49 | 120 | 110 | 42 |
| 9 | 7 | 65 | 56 | 40 | 120 | 114 | 30 |
| C2 | FC-732 | 71 | 69 | 46 | 125 | 109 | 57 |

The data in TABLE 2 show that all of the fluorocopolymers containing short chain fluoroalkyl groups performed comparably to the comparative fluorocopolymer containing longer perfluoroalkyl groups.

### Examples 10-16 and Comparative Example C3

In this series of experiments, the same general procedure was followed as with the tests run in TABLE 2, using the same aluminum test wafer, except in this series, fluorocopolymers were applied at 0.1 % solids from HFE-7100. Also, in this series, only static contact angles were measured. Results are presented in TABLE 3.

**TABLE 3**

| **Example** | **Fluorocopolymer** | **Static Contact Angle:** | |
|---|---|---|---|
| | | ***n*-C**_{**16**}**H**_{**34**} | **water** |
| 10 | 1 | 48 | 106 |
| 11 | 2 | 43 | 97 |
| 12 | 3 | 53 | 103 |
| 13 | 4 | 55 | 114 |
| 14 | 5 | 57 | 114 |
| 15 | 6 | 65 | 113 |
| 16 | 7 | 63 | 107 |
| C3 | FC-732 | 57 | 109 |

The data in TABLE 3 show that all of the fluorocopolymers containing short chain fluoroalkyl groups performed comparably to the comparative fluorocopolymer containing longer perfluoroalkyl groups.

### Examples 17-23 and Comparative Example C4

In this series of experiments, the same general procedure was followed as with the tests run in TABLE 1 using the same 1#310 3M Al₂O₃-TiC Ceramics test wafer and this time with Fluorocopolymers 1-9. However, this time all fluorocopolymers were applied at 0.1 % solids from HFE-7100. Results are presented in TABLE 4.

**TABLE 4**

| **Ex.** | **Fluorocopolymer** | ***n*-hexadecane** | | **water** | |
|---|---|---|---|---|---|
| | | **Adv** | **Rec** | **Stat** | **Rec** |
| 17 | 1 | 66 | 41 | 116 | 63 |
| 18 | 2 | N/M | N/M | 104 | 67 |
| 19 | 3 | 38 | 30 | 95 | 50 |
| 20 | 4 | 66 | 44 | 120 | 66 |
| 21 | 5 | 65 | 42 | 120 | 56 |
| 22 | 6 | 65 | 49 | 116 | 58 |
| 23 | 7 | 59 | 44 | 115 | 52 |
| C4 | FC-732 | 63 | 39 | 125 | 45 |

| | | | | | |
|---|---|---|---|---|---|
| * Not measured | | | | | |

The data in TABLE 4 show that all of the fluorocopolymers containing short chain fluoroalkyl groups performed comparably to the comparative fluorocopolymer containing longer perfluoroalkyl groups.

### Examples 24 and 25

Static contact angles were measured for glass slides treated with Fluorocopolymer 8 (Mₙ = 10400 and M_{w} = 22300) and Fluorocopolymer 9 (Mₙ = 6180 and M_{w} = 16700) to determine the effect of polymer molecular weight on contact angle. Both fluorocopolymers had the same theoretical composition of 99.95/0.05 FBMA/AA.

The static contact angle test procedure was slightly modified for this test series. A clean glass microscope slide was dipped into the test fluorocopolymer solution for 10 seconds, and the treated slide was removed and allowed to air dry for about one minute. Then three drops of either n-hexadecane (about 0.02 mL) or deionized water were applied to the treated slide surface using a 1 mL syringe and static contact angles were measured using the earlier described Rame-Hart static contact angle goniometer.

Results are presented in TABLE 5. Reported values are the averages of measurements for each of the three drops and represent four replicates for Example 24 and two replicates for Example 25.

**TABLE 5**

| **Example** | **Fluorocopolymer** | **Static Contact Angle:** | |
|---|---|---|---|
| | | ***n*-C**_{**16**}**H**_{**34**} | **water** |
| 24 | 10 | 66 | 121 |
| 25 | 11 | 64 | 118 |

The data in TABLE 5 show that Fluorocopolymer 8, having a higher molecular weight, produced slightly higher static contact angles than did Fluorocopolymer 9., having a lower molecular weight.

### Example 26 and 27

For Examples 26 and 27, respectively, two fluorocopolymers were made at about 30% solids in a highly fluorinated solvent consisting of 100% methyl perfluorobutyl ether (HFE-71 00) by copolymerizing CF₃CF₂CF₂CH₂OC(O)C(CH₃)=CH₂ (FBMA) with 1.7% and 10% acrylic acid (AA) using LUPEROX™ 26-M50 initiator. The charges (grams) used to make each fluorocopolymer are shown below in TABLE 6.

**TABLE 6**

| **Charge (g):** | **Example 32 (1.7/98.3 FBMA/AA)** | **Example 33 (10/90 FBMA/AA)** |
|---|---|---|
| FBMA | 141.00 | 126.90 |
| AA | 2.48 | 14.10 |
| LUPEROX™ 26-M50 | 5.67 | 5.64 |
| HFE-7100 | 329.00 | 329.00 |

For each fluorocopolymer, the charges were placed in a reaction vessel and purged four times with dry nitrogen to minimize the residual oxygen level. The vessel was sealed and the resulting mixture was heated with agitation at 80°C for 23 hours to complete the polymerization reaction. After reaction, the vessel was opened and the polymer solution was examined for homogeneity.

For the polymer of Example 26, a clear, thick, homogeneous polymer solution formed in the highly fluorinated solvent. For the polymer of Example 27, a tough white solid separated out of solution from the highly fluorinated solvent. Thus, for application from highly fluorinated solvents, which are preferred for safety and environmental reasons, it is advantageous to use fluorocopolymers containing less than 10% copolymerized acrylic acid to insure maximum fluorocopolymer solubility.

## Claims

1. A coating composition comprising
a partially fluorinated solvent, and
a fluoroalkyl (meth)acrylate/(meth)acrylic acid copolymer, wherein said fluoroalkyl group has 6 carbon atoms or fewer and wherein said copolymer comprises 5 wt.% or less of (meth)acrylic acid when said fluoroalkyl group is a perfluoroalkyl group.

2. The coating composition of claim 1 wherein said copolymer is of the formula: 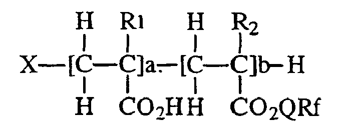 wherein the sum of a + b is an number such that the compound is polymeric;
R₁ is hydrogen, or methyl group
R₂ is a straight chain or branched chain alkyl containing 1 to about 4 carbon atoms;
Q is a covalent bond or an organic divalent linking group,
R_{f} is a fluoroaliphatic group that comprises a fully fluorinated terminal group having less than 6 carbon atoms;
and X is a hydrogen atom or a group derived from a free radical initiator;

3. The composition of claim 2 where R_{f} has 3 or 4 carbon atoms.

4. The composition of claim 2 wherein Q is -CH₂-.

5. The composition of claim 1 wherein said partially fluorinated solvent is selected from hydrochlorofluorocarbons, hydrofluorocarbons and hydrofluoroethers.

6. The composition of claim 1 wherein said fluorinated ether is of the formula
(R-O)ₓ-R'_{f} (I)
wherein x is about I or 2; R represents an alkyl group having about I to 4 carbon atoms; and R'_{f} represents a fluoroaliphatic group.

7. A film comprising the cured coating composition of any of claims 1 to 6.

8. The film of claim 7 having a refractive index of less than 1.5.

9. A slider for use in a magnetic media disk, the slider bearing a thin film of the cured coating composition of any of claims 1 to 6.

10. A pellicle comprising a cured film of the coating composition of any of claims 1 to 6, said film secured to a frame.

11. An antimigration coating to prevent lubricant from spreading beyond an area of frictional contact comprising the coating composition of any of claims 1 to 6, said coating composition being disposed in a predetermined pattern near the area of frictional contact.

12. The composition of any of claims 1-6 comprising 2 wt.% or less of (meth)acrylic acid.

## Patentansprüche

1. Beschichtungszusammensetzung, umfassend
ein teilfluoriertes Lösungsmittel und
ein Fluoralkyl(meth)acrylat/(Meth)acrylsäureCopolymer, wobei die Fluoralkylgruppe 6 Kohlenstoffatome oder weniger aufweist und das Copolymer 5 Gew.-% oder weniger (Meth)acrylsäure umfasst, wenn es sich bei der Fluoralkylgruppe um eine Perfluoralkylgruppe handelt.

2. Beschichtungszusammensetzung nach Anspruch 1, wobei das Copolymer die Formel: 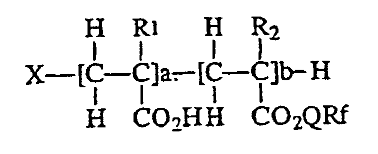 aufweist, wobei die Summe von a + b eine solche Zahl ist, dass die Verbindung polymer ist;
R₁ für Wasserstoff oder eine Methylgruppe steht;
R₂ für geradkettiges oder verzweigtkettiges Alkyl mit 1 bis etwa 4 Kohlenstoffatomen steht;
Q für eine kovalente Bindung oder eine organische zweiwertige Verknüpfungsgruppe steht;
R_{f} für eine fluoraliphatische Gruppe steht, die eine vollfluorierte Endgruppe mit weniger als 6 Kohlenstoffatomen umfasst;
und X für ein Wasserstoffatom oder eine von einem radikalischen Initiator abgeleitete Gruppe steht.

3. Zusammensetzung nach Anspruch 2, wobei R_{f} 3 oder 4 Kohlenstoffatome aufweist.

4. Zusammensetzung nach Anspruch 2, wobei Q für -CH₂- steht.

5. Zusammensetzung nach Anspruch 1, wobei das teilfluorierte Lösungsmittel aus Chlorfluorkohlenwasserstoffen, Fluorkohlenwasserstoffen und Hydrofluorethern ausgewählt ist.

6. Zusammensetzung nach Anspruch 1, wobei der fluorierte Ether die Formel
(R-O)ₓ-R'_{f} (I)
aufweist, wobei x etwa 1 oder 2 ist, R für eine Alkylgruppe mit etwa 1 bis 4 Kohlenstoffatomen steht und R'_{f} für eine fluoraliphatische Gruppe steht.

7. Folie, umfassend die gehärtete Beschichtungszusammensetzung gemäß einem der Ansprüche 1 bis 6.

8. Folie nach Anspruch 7 mit einem Brechungsindex von weniger als 1,5.

9. Slider zur Verwendung in einer Magnetspeicherplatte, wobei der Slider eine dünne Folie aus der gehärteten Beschichtungszusammensetzung gemäß einem der Ansprüche 1 bis 6 aufweist.

10. Membran, umfassend eine gehärtete Folie aus der Beschichtungszusammensetzung gemäß einem der Ansprüche 1 bis 6, wobei die Folie an einem Rahmen befestigt ist.

11. Antimigrationsbeschichtung zur Verhinderung des Ausbreitens von Schmiermittel über eine Reibungskontaktfläche hinaus, umfassend die Beschichtungszusammensetzung gemäß einem der Ansprüche 1 bis 6, wobei die Beschichtungszusammensetzung in einem vorbestimmten Muster in der Nähe der Reibungskontaktfläche angeordnet ist.

12. Zusammensetzung nach einem der Ansprüche 1-6, umfassend 2 Gew.-% oder weniger (Meth)acrylsäure.

## Revendications

1. Composition de revêtement comprenant
un solvant partiellement fluoré, et
un copolymère de (méth)acrylate de fluoroalkyle/acide (méth)acrylique, dans lequel ledit groupe fluoroalkyle renferme 6 atomes de carbone ou moins et où ledit copolymère comprend 5 % en poids ou moins d'acide (méth)acrylique lorsque ledit groupe fluoroalkyle est un groupe perfluoroalkyle.

2. Composition de revêtement selon la revendication 1, dans laquelle ledit copolymère est de formule : 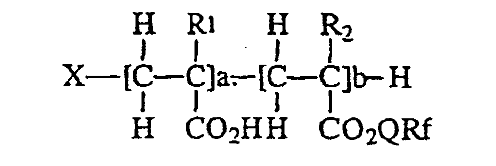 dans laquelle la somme de a + b est un nombre tel que le composé soit polymère ;
R₁ est un hydrogène ou un groupe méthyle ;
R₂ est un alkyle à chaîne linéaire ou à chaîne ramifiée renfermant de 1 à environ 4 atomes de carbone ;
Q est une liaison covalente ou un groupe de liaison bivalent organique ;
R_{f} est un groupe fluoroaliphatique renfermant un groupe terminal totalement fluoré renfermant moins de 6 atomes de carbone ;
et X est un atome d'hydrogène ou un groupe dérivé d'un amorceur de radicaux libres.

3. Composition selon la revendication 2, dans laquelle R_{f} renferme 3 ou 4 atomes de carbone.

4. Composition selon la revendication 2, dans laquelle Q est -CH₂-.

5. Composition selon la revendication 1, dans laquelle ledit solvant partiellement fluoré est choisi parmi des hydrochlorofluorocarbones, des hydrofluorocarbones et des hydrofluoroéthers.

6. Composition selon la revendication 1, dans laquelle ledit éther fluoré est de formule
(R-O)ₓ-R'_{f} (I)
dans laquelle x vaut environ 1 ou 2 ; R représente un groupe alkyle renfermant d'environ 1 à 4 atomes de carbone ; et R'_{f} représente un groupe fluoro aliphatique.

7. Film comprenant la composition de revêtement durcie selon l'une quelconque des revendications 1 à 6.

8. Film selon la revendication 7, présentant un indice de réfraction inférieur à 1,5.

9. Coulisse destinée à être utilisée dans un disque de support magnétique, la coulisse portant un film mince de la composition de revêtement durcie selon l'une quelconque des revendications 1 à 6.

10. Pellicule comprenant un film durci de la composition de revêtement selon l'une quelconque des revendications 1 à 6, ledit film étant fixé à un cadre.

11. Revêtement antimigration destiné à empêcher que le lubrifiant s'étale au-delà d'une zone de contact de frottement, comprenant la composition de revêtement selon l'une quelconque des revendications 1 à 6, ladite composition de revêtement étant disposée en une configuration prédéterminée à proximité de la zone de contact de frottement.

12. Composition selon l'une quelconque des revendications 1 à 6, comprenant 2 % en poids ou moins d'acide (méth)acrylique.
